Europäisches Patentamt

European Patent Office

Office européen des brevets

(19)

(11) **EP 0 808 835 A1**

(12) **EUROPEAN PATENT APPLICATION**

(43) Date of publication:
26.11.1997 Bulletin 1997/48

(21) Application number: 97890092.6

(22) Date of filing: 21.05.1997

(51) Int. Cl.$^6$: **C07D 215/18**, C07D 471/06,
C07C 69/76, C07B 57/00
// (C07D471/06, 221:00,
221:00)

(84) Designated Contracting States:
AT BE CH DE DK ES FI FR GB GR IE IT LI LU MC
NL PT SE

(30) Priority: 21.05.1996 HU 9601362

(71) Applicant:
CHINOIN
Gyògyszer és Vegyészeti Termékek Gyára RT.
H-1045 Budapest IV (HU)

(72) Inventors:
• Balint, Jozsef
Damjanich u. 2. (HU)

• Fetter, György
Landorhegyi u. 24/A,I/5 (HU)
• Fogassy, Elemér
Ordögorom u. 20. (HU)
• Friesz, Antal
Buvar u. 1. (HU)
• Gajary, Antal
Bölöni Gy. u. 15/B (HU)

(74) Representative:
Schwarz, Albin, Dr.
Kopecky & Schwarz
Patentanwälte et al
Wipplingerstrasse 32/22
1010 Wien (AT)

(54) **Process for the preparation of optically active compounds**

(57) Process for the resolution of 6-fluoro-1,2,3,4-tetrahydro-2-methyl-quinoline using L-(-)-di-para-toluoyl-tartaric acid as resolving agent.

## Description

This invention relates to the new optically active intermediate of formula (I) and its salts, the new intermediate of formula (VI), the optically pure compound of formula (II) and its salts, and the novel preparation process to obtain compounds of formula (I) and (II) and their salts.

The racemic 6-fluoro-1,2,3,4-tetrahydro-2-methyl-quinoline and the racemic 9-fluoro-6,7-dihydro-5-methyl-1-oxo-1$H$,5$H$-pyrido[3,2,1-$i,j$]quinoline-2-carboxylic acid and its salts are known from the literature / German Patent Application publ. No. 2.264163 and French Patent Application publ. No. 2453647/. The latter pyridoquinoline derivative is used as an antibacterial agent in the human and veterinary medicine and in the course of cultivation of plants.

Our aim was to prepare the optically active compound of formula (II) -which possesses more favourable antibacterial properties than the above mentioned racemic pyridoquinoline - by an improved process and in higher optical purity.

We found that the resolution of the racemic 6-fluoro-1,2,3,4-tetrahydro-2-methyl-quinoline intermediate can be effected by applying L-(-)di-para-toluoyl-tartaric acid. Resolution is carried out by reacting the racemic 6-fluoro-1,2,3,4-tetrahydro-2-methyl-quinoline with L-(-)di-para-toluoyl-tartaric acid, the resulting acid salt of formula (VI) is then isolated from the reaction mixture by a method known per se, if desired, the salt is purified, occasionally the acid salt of formula (VI) is recovered from the purification mother liquor, and if desired purified, finally the (-)-6-fluoro-1,2,3,4-tetrahydro-2-methyl-quinoline of formula (I) is liberated from the acid salt of formula (VI), and if desired, transformed into another salt.

Performing the resolution with one equimolecular or less than equimolecular amount of di-para-toluoyl-tartaric acid, with or without the addition of an achiral acid, the diastereomers can be separated from various solvents, i.e. strongly polar solvents, such as water, methanol, ethanol, acetonitrile, ethyl acetate, acetone, or less polar solvents, such as chloroform, toluene or from the mixtures of the above, or by applying water and a water non-miscible solvent, together.

We found as a very favourable method to add 0.5 molar equivalent of di-para-toluoyl-tartaric acid and concentrated aqueous hydrochloric acid to the racemic 6-fluoro-1,2,3,4-tetrahydro-2-methyl-quinoline base, preferably in methanol, and filter off the precipitated crystals of the novel L-(-)-di-para-toluoyl-tartrate acid salt of formula (VI). The salt is then taken up in methanol, the resulting suspension is heated under reflux, after cooling the crystals are filtered off, dried and finally the base is liberated. The thus obtained (-)-enantiomer of formula (I) has a very high optical purity. Liberation and isolation of the base from the diastereomer salt is effected by adjusting the pH of the suspension to alkaline, extracting the free (-)-enantiomer with dichloromethane, and evaporating the organic solution.

Following evaporation of the aqueous mother liquors a mixture of the (+)-enantiomer and of the unreacted racemic 6-fluoro-1,2,3,4-tetrahydro-2-methyl-quinoline is obtained by extraction. From this mixture both compounds may be recovered by various ways.

One possibility is to transform the racemic 6-fluoro-1,2,3,4-tetrahydro-2-methyl-quinoline, which remained in the mother liquors, into its hydrochloride salt and remove the (+)-6-fluoro-1,2,3,4-tetrahydro-2-methyl-quinoline from the mixture by distillation in vacuo, occasionally the hydrochloride salt formation and the distillation may repeatedly be performed, both with the distilled material and with the distillation residue, one or more times.

According to another method the racemic 6-fluoro-1,2,3,4-tetrahydro-2-methyl-quinoline and the (+)-6-fluoro-1,2,3,4-tetrahydro-2-methyl-quinoline remaining dissolved in the mother liquor are separated from each other by creating conditions where their partitions between the solid and the solution phases are different.

According to this method either the (+)-6-fluoro-1,2,3,4-tetrahydro-2-methyl-quinoline is taken into the solid phase, by lowering the temperature, or the racemic 6-fluoro-1,2,3,4-tetrahydro-2-methyl-quinoline is precipitated, by adding aqueous hydrochloric acid to the mixture. Carrying out the above procedures, if needed repeatedly, the optically pure (+)-6-fluoro-1,2,3,4-tetrahydro-2-methyl-quinoline and the unchanged racemic compound may be obtained separately. The racemic compound may then be resolved again, whereas the (+) enantiomer may be racemized. The above two methods significantly improve the effectiveness of the present process and owing to them the use of further foreign chiral reagents can be avoided.

The optically active compound of formula (I) is then transformed into the optically pure compound of general formula (II) by applying the method described below.

The (-)-6-fluoro-1,2,3,4-tetrahydro-2-methyl-quinoline of formula (I) is reacted with the dialkyl (alkoxymethylene)malonate of general formula (III) -wherein R is a $C_{1-4}$ alkyl group - and the resulting diester of general formula (IV) -wherein R is a $C_{1-4}$ alkyl group - is cyclized into the carboxylic acid ester of general formula (V) - wherein R has the same meaning as above - which is then transformed by hydrolysis into the compound of formula (II) and, if desired a salt of the compound of formula (II) may be formed.

Of the compounds of general formula (III) the use of diethyl (ethoxymethylene)malonate is the most advantageous. The ethanol which is formed in the condensation reaction is distilled off during the reaction, the temperature of the reaction is favourably 110 to 130 °C.

Ring closure of the compounds of general formula (IV) may preferably be performed in the presence of polyphosphoric acid, which is added to the cooled (20°C) reaction mixture, the temperature is then raised to 100 to 120°C. Hydrolysis of the obtained esters of general formula (V) is effected at reflux temperature and the resulting compound of formula (II), which precipitates from the reaction mixture after cooling, may be separated from the mixture by a method known per se. The obtained raw product of formula (II) may be purified by recrystallization, preferably e.g. from dimethyl formamide.

In the course of the preparation of the compound of formula (II) the intermediates of general formulae (IV) and (V) are preferably not isolated.

During the above multi-step synthesis, surprisingly, the optical purity does not deteriorate, racemization and degradation do not take place, thus the targeted compound of formula (II) can be prepared in an enantiomeric excess of 98%, at the least. The optical purity plays important role in the biological behaviour of optically active compounds /Chirality **5**, p. 350 to 355 (1993)/.

Further details of our invention are shown by the examples below, without limiting our claims to the examples.

## EXAMPLES

### Example 1.

a.) 10.00 g of the racemic 6-fluoro-1,2,3,4-tetrahydro-2-methyl-quinoline, previously distilled in vacuo, was dissolved in 12.5 ml of methanol, to the solution 11.70 g of di-para-toluoyl-tartaric acid dissolved in 25 ml of methanol was added at 50°C, and 2.50 ml of 37 % hydrochloric acid solution (d=1.18 g/cm$^3$) was poured onto the mixture. Upon cooling, inoculation and scraping crystallization started from the solution. The resulting dense suspension was cooled to 20°C, stirred for 10 minutes, the crystals were collected by filtration, washed with 4x3 ml of 10°C methanol. The mother liquor (AO) was saved. Thus, 12.9 g of wet (-)-6-fluoro-1,2,3,4-tetrahydro-2-methyl-quinoline L-(-)-di-para-toluoyl-tartrate acid salt was obtained.

b.) 12.9 g of the wet crystalline salt obtained above was taken up in 25 ml of methanol, the resulting suspension was refluxed for 10 minutes, the crystals were collected and washed with 20°C methanol. After drying the wet salt under infrared lamp 9.6 g of(-)-6-fluoro-1,2,3,4-tetrahydro-2-methyl-quinoline L-(-)-di-para-toluoyl-tartrate acid salt was obtained. Mp.: 171-173°C, $\alpha_D^{RT}$]= -126.9° (c=1, methanol).

c.) 9.4 g of the diastereomer salt obtained above was suspended in 40 ml of water, made alkaline by adding 1 g of sodium hydroxide and 0.85 ml of 25% ammonium hydroxide solution, and after stirring for 15 minutes with 10 ml of dichloromethane the phases were separated. The aqueous phase was extracted with additional 2x 10 ml of dichloromethane. The aqueous phase was saved.

The dichloromethane phases were united, dried over sodium sulfate, filtered and the dichloromethane was distilled off in vacuo. Thus, 2.68 g of (-)-6-fluoro-1,2,3,4-tetrahydro-2-methyl-quinoline was obtained as an oil, which solidified following inoculation; $\alpha_D^{RT}$] = -67.3° (c=1, ethanol).

d.) From the mother liquor (AO) of the resolution the methanol was distilled off in vacuo. The distillation residue (12.5 g) was taken up in 30 ml of water, made alkaline with 1.85 g of sodium hydroxide and 2.10 ml of 25 % ammonium hydroxide solution. The mixture was stirred with 10 ml of dichloromethane for 15 minutes, and the phases were separated. The aqueous phase was extracted with additional 2x10 ml of dichloromethane then it was saved.
The dichloromethane phases were united, dried over sodium sulfate, filtered, the solvent was removed in vacuo to obtain 6.80 g of an oil, consisting of (+)-6-fluoro-1,2,3,4-tetrahydro-2-methyl-quinoline. Following inoculation the oil solidified; $\alpha_D^{RT}$] = +28.1° (c=1, ethanol).

e.) From the mother liquor (A1) obtained at the purification of the diastereomer salt, the methanol was distilled off in vacuo, the residue, 1.4 g was suspended in 15 ml of water, made alkaline with 0.15 g of sodium hydroxide and 0.15 ml of 25 % ammonium hydroxide solution. The mixture was stirred for 15 minutes with 10 ml of dichloromethane, and the phases were separated. The aqueous phase was extracted with 2x10 ml of dichloromethane then it was saved. The dichloromethane phases were united, dried over sodium sulfate, filtered, and the dichloromethane was removed in vacuo. The residual oil, 0.50 g, which following inoculation may stiffen into oily crytals, consists mainly of (-)-6-fluoro-1,2,3,4-tetrahydro-2-methyl-quinoline; $\alpha_D^{RT}$] = -27.7° (c=1, ethanol).

f.) The aqueous phases, saved above, were united, traces of the dichloromethane were removed in vacuo. The solution was filtered, acidified with 37% hydrochloric acid solution to pH=1 (8 ml). In case the di-para-toluoyl-tartaric acid precipitated in the form of an oil, the mixture was stirred until the oil crystallized.
The crystals were collected, washed with water, dried under infrared lamp. Thus, 9.47 g of white, crystalline di-para-toluoyl-tartaric acid was recovered; $\alpha_D^{RT}$] = -131.9° (c=1, ethanol).

### Example 2.

1.00 g of (-)-6-fluoro-1,2,3,4-tetrahydro-2-methyl-quinoline $[\alpha]_D^{RT}$ = -70.0° (enantiomeric excess >98%, c=1, ethanol) was heated at 120°C with 1.56 g of diethyl (ethoxymethylene)malonate. After the ethanol distillation has ceased, the reaction apparatus was kept under vacuo for 5 minutes, using an ejector-jet pump. The hot, 120 °C reaction mixture was then cooled to room temperature, 1.50 g of polyphosphoric acid was added, and the mixture was stirred and heated at 110 °C for 5 hours and 30 mins. Following the addition of 3 ml of water it was further heated at 110 °C under reflux conditions for another 2 hours. The mixture was cooled down to room temperature and the crystals of the resulting thick suspension were collected, washed subsequently with 5 x 1 ml of water and 3 x 0.25 ml of ethanol. The wet raw product was recrystallized from 2 ml of hot dimethyl formamide, the crystals were collected, washed subsequently with 2 x 0.25 ml of dimethyl formamide, 3 x 1 ml of water and 2 x 0.25 ml of ethanol, then they were dried to constant mass. Thus 1.2 g (75.9 %) white crystals of (-)-9-fluoro-6,7-dihydro-5-methyl-1-oxo-1$H$,5$H$-pyrido[3,2,1-$i$,$j$]quinoline-2-carboxylic acid /(-)-flumequin/ was obtained, enantiomeric excess: >98%, $[\alpha]_D^{RT}$ = -48.9° (c= 0.24, dimethyl formamide), mp.: 245-248°C.

## Example 3.

34.6 g (209.4 mmol) of 6-fluoro-1,2,3,4-tetrahydro-2-methyl-quinoline $[\alpha]_D^{26}$= +31.75 ° (c = 1, ethanol) was dissolved in 35 ml of ethanol. Under cooling 9.6 ml (114.9 mmol) of 37% hydrochloric acid (d = 1.18) was added. The solvent was removed in vacuo, the residue was distilled. The fraction obtained around 130 °C /1.6 kPa was rich in the (+) enantiomer. This fraction, 14.22 g (86.1 mmol, 41.1% ) $[\alpha]_D^{26}$ = +41.9 ° (c = 1, ethanol), was an oil which stiffened into oily crystals following inoculation.

To the solid residue of the distillation 70 ml of water and 5.0 g (125 mmol) of sodium hydroxide was added and the solution was extracted with 3 x 20 ml of dichloromethane. The combined organic phases were dried over sodium sulfate, filtered and evaporated. The residue was an oil, 19.04 g (115.2 mmol, 55.0%), $[\alpha]_D^{26}$ = +22.4° (c = 1, ethanol). The oil stiffened into oily crystals following inoculation.

## Example 4.

To 66.0 g (399.5 mmol) of 6-fluoro-1,2,3,4-tetrahydro-2-methyl-quinoline $[\alpha]_D^{20}$= +7.2° (c = 1, ethanol) 200 ml of water and 35 ml of 37% hydrochloric acid (419.0 mmol, d = 1.18) were added and the mixture was heated until dissolution. From the clear solution the crystallization started on cooling and scraping. The resulting suspension was cooled down to 20 °C, the crystals were collected, washed with 3 x 15 ml of 0°C water. The thus obtained wet crystals of 6-fluoro-1,2,3,4-tetrahydro-2-methyl-quinoline hydrochloride were taken up in 150 ml of water. To the suspension 13.0 g (325.0 mmol) of sodium hydroxide was added and the mixture was extracted with 3 x 50 ml of dichloromethane. The organic phases were combined, dried over sodium sulfate and filtered. Dichloromethane was removed in vacuo to obtain 48.0 g (290.5 mmol, 72.7 %) of 6-fluoro-1,2,3,4-tetrahydro-2-methyl-quinoline base $[\alpha]_D^{20}$= +1.0° (c = 1, ethanol).

To the mother liquor of the hydrochloride salt 7.0 g (175.0 mmol) of sodium hydroxide was added and the mixture was extracted with 3 x 50 ml of dichloromethane. The organic layers were combined, dried over sodium sulfate. Dichloromethane was removed in vacuo to obtain 13.9 g (84.1 mmol, 21.1%) of 6-fluoro-1,2,3,4-tetrahydro-2-methyl-quinoline base $[\alpha]_D^{20}$= +25.8° (c = 1, ethanol).

## Example 5.

By a method similar as described in Example 1., starting from 130.0 g (786.7 mmol) of 6-fluoro-1,2,3,4-tetrahydro-2-methyl-quinoline with optical rotation $[\alpha]_D^{20}$ = +29.7° (c = 1, ethanol), by preparing and collecting the crystals of the 6-fluoro-1,2,3,4-tetrahydro-2-methyl-quinoline hydrochloride, and liberating the base, 66.5 g (402.5 mmol, 51.2 %) of 6-fluoro-1,2,3,4-tetrahydro-2-methyl-quinoline base $[\alpha]_D^{20}$ = +7.2° (c = 1, ethanol) was obtained, whereas by working up the mother liquor of the hydrochloride salt, 57.2 g (346.2 mmol, 44.0 %) of $[\alpha]_D^{20}$ = +57.4° (c = 1, ethanol) 6-fluoro-1,2,3,4-tetrahydro-2-methyl-quinoline base was obtained.

## Example 6.

11.5 g (69.6 mmol) of 6-fluoro-1,2,3,4-tetrahydro-2-methyl-quinoline base $[\alpha]_D^{20}$ = + 35.4° (c = 1, ethanol) was cooled to 0 - 5 °C, and the oil was seeded with the crystals of the (+)-6-fluoro-1,2,3,4-tetrahydro-2-methyl-quinoline enantiomer. The resulting crystalline mass was crushed, it was placed on a glass filter previously cooled to approximately 0 °C, and it was filtered, while the temperature was allowed to gradually raise to ambient (approx. 22°C). The material collected on the filter, 1.2 g (7.3 mmol, 10.4%), was the white crystalline 6-fluoro-1,2,3,4-tetrahydro-2-methyl-quinoline base $[\alpha]_D^{20}$ = +64.8° (c = 1, ethanol), whereas the filtrate, 9.9 g (59.9 mmol, 86.1%) consisted of the oily 6-fluoro-1,2,3,4-tetrahydro-2-methyl-quinoline base $[\alpha]_D^{20}$ = +32.8° (c = 1, ethanol).

## Example 7.

By a method similar as described in Example 3., starting from 14.5 g (87.8 mmol) of 6-fluoro-1,2,3,4-tetrahydro-2-methyl-quinoline base $[\alpha]_D^{20}$ = +40.3° (c = 1, ethanol), the material collected on the filter, 2.4 g (14.5 mmol, 16.6 %) was the white, crystalline 6-fluoro-1,2,3,4-tetrahydro-2-methyl-quinoline base, $[\alpha]_D^{20}$ = +65.2° (c = 1, ethanol). The filtrate, 12.0 g (72.6 mmol, 82.8%) consisted of the oily $[\alpha]_D^{20}$ = +35.4° (c = 1, ethanol) 6-fluoro-1,2,3,4-tetrahydro-2-methyl-quinoline base.

## Example 8.

By a method similar as described in Example 3., starting from 22.0 g (133.2 mmol) of 6-fluoro-1,2,3,4-tetrahydro-2-methyl-quinoline base $[\alpha]_D^{20} = + 48.8°$.(c = 1, ethanol), the material collected on the filter, 6.5 g (39.3 mmol, 29.5%) was the white, crystalline 6-fluoro-1,2,3,4-tetrahydro-2-methyl-quinoline base $[\alpha]_D^{20} = +65.8°$ (c = 1, ethanol). The filtrate, 15.1 g (91.4 mmol, 68.6%) consisted of the oily $[\alpha]_D^{20} = +40.3°$ (c = 1, ethanol) 6-fluoro-1,2,3,4-tetrahydro-2-methyl-quinoline base.

## Example 9.

55.0 g (332.9 mmol) of oily $[\alpha]_D^{20} = +57.4°$ (c = 1, ethanol) 6-fluoro-1,2,3,4-tetrahydro-2-methyl-quinoline base was seeded at room temperature (approx. 22°C) with the crystals of the (+)-6-fluoro-1,2,3,4-tetrahydro-2-methyl-quinoline enantiomer. (In case the crystallization did not start, the mixture was cooled to 10 to 15°C.) The mixture was allowed to crystallize at room temperature for 1 hour, then it was filtered. The material collected on the filter, 31.9 g (193.1 mmol, 58.0%), was the white crystalline 6-fluoro-1,2,3,4-tetrahydro-2-methyl-quinoline base $[\alpha]_D^{20} = +65.4°$ (c = 1, ethanol). The filtrate, 22.9 g (138.6 mmol, 41.6%) consisted of the oily 6-fluoro-1,2,3,4-tetrahydro-2-methyl-quinoline base $[\alpha]_D^{20} = +48.8°$ (c = 1, ethanol).

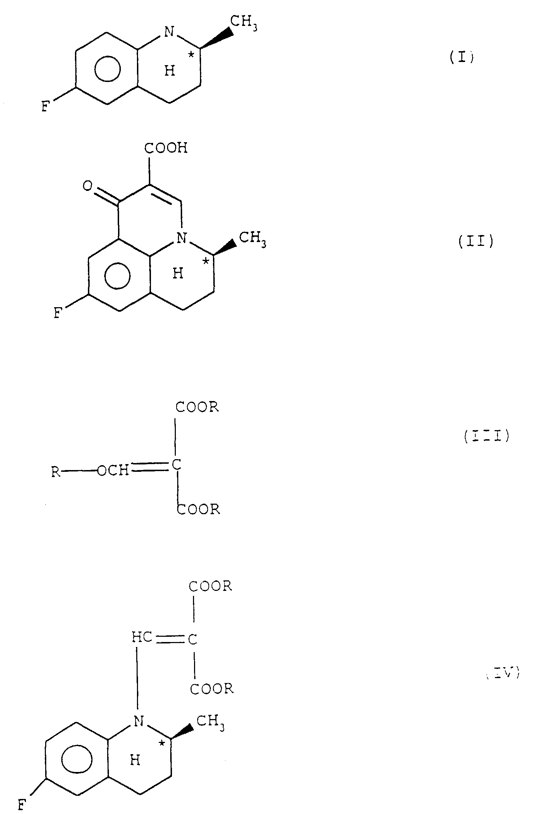

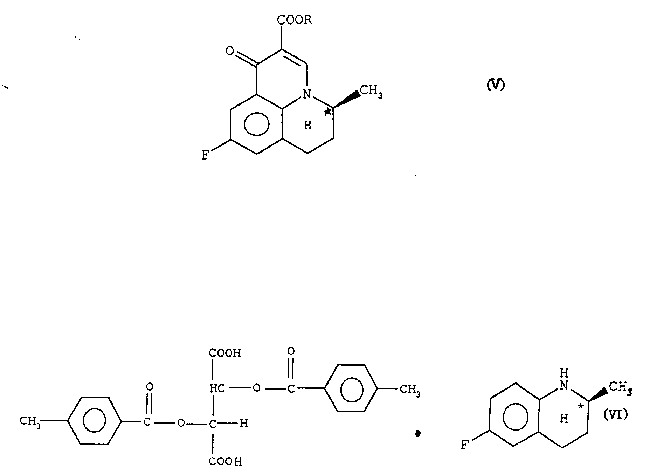

## Claims

1. Process for the preparation of the (-)-6-fluoro-1,2,3,4-tetrahydro-2-methyl-quinoline of formula (I) and its salts **characterized by** that the racemic 6-fluoro-1,2,3,4-tetrahydro-2-methyl-quinoline or its salt is resolved with L-(-)-di-para-toluoyl-tartaric acid or its salt.

2. Process, according to claim 1., for the preparation of the (-)-6-fluoro-1,2,3,4-tetrahydro-2-methyl-quinoline of formula (I) and its salts **characterized by** that the racemic 6-fluoro-1,2,3,4-tetrahydro-2-methyl-quinoline is reacted with L-(-)-di-para-toluoyl-tartaric acid, the acid salt of formula (VI) is isolated from the reaction mixture by a method known per se, if desired it is purified, occasionally the acid salt of formula (VI) is isolated also from the purification mother liquor, and is purified, and from the acid tartrate salt of formula (VI) the (-)-6-fluoro-1,2,3,4-tetrahydro-2-methyl-quinoline of formula (I) is liberated, and if desired transformed into an other salt.

3. Process, according to claim 1., **characterized by** that the L-(-)-di-para-toluoyl-tartaric acid is applied in half molar equivalent amount.

4. Process, according to claim 1., **characterized by** that the reaction is carried out in the presence of a solvent.

5. Process, according to claim 1., **characterized by** that L-(-)-di-para-toluoyl-tartaric acid and hydrochloric acid are applied together in altogether one molar equivalent amount.

6. Process, according to claim 1., **characterized by** that the racemic 6-fluoro-1,2,3,4-tetrahydro-2-methyl-quinoline is dissolved in a water-miscible organic solvent, to this solution the L-(-)-di-para-toluoyl-tartaric acid dissolved in a water-non-miscible organic solvent and aqueous hydrochloric acid are added, and the acid salt of formula (VI) formed from the compound of formula (I) and L-(-)-di-para-toluoyl-tartaric acid is isolated by filtration, purified by recrystallization, and the compound of formula (I) is liberated from the salt on the effect of an aqueous base and it is isolated from the reaction mixture.

7. Process, according to claim 1., **characterized by** that the racemic 6-fluoro-1,2,3,4-tetrahydro-2-methyl-quinoline, remaining in the mother liquors, is transformed into its hydrochloride salt and the (+)-6-fluoro-1,2,3,4-tetrahydro-2-methyl-quinoline is separated from it by distilling it off in vacuo, and occasionally the hydrochloride salt formation and the distillation may be carried out repeatedly, from both the distilled material and the distillation residue, one or more times.

8. Process, according to claim 1., **characterized by** that the racemic 6-fluoro-1,2,3,4-tetrahydro-2-methyl-quinoline and the (+)-6-fluoro-1,2,3,4-tetrahydro-2-methyl-quinoline, remaining in the mother liquors, are separated from their mixture by creating conditions where their partitions between the solid and the solution phases are different.

9. Process, according to claim 8., **characterized by** that by lowering the temperature the (+)-6-fluoro-1,2,3,4-tetrahydro-2-methyl-quinoline is taken into the solid phase.

10. Process, according to claim 8., **characterized by** that by the addition of aqueous hydrochloric acid the racemic 6-fluoro-1,2,3,4-tetrahydro-2-methyl-quinoline is taken into the solid phase, in the form of its hydrochloride salt.

11. Process for the preparation of the (-)-9-fluoro-6,7-dihydro-5-methyl-1-oxo-1*H*,5*H*-pyrido[3,2,1-*i,j*]quinoline-2-carboxylic acid of formula (II), **characterized** by that the (-)-6-fluoro-1,2,3,4-tetrahydro-2-methyl-quinoline of formula (I) is reacted with the dialkyl (alkoxymethylene)malonate of general formula (III) - wherein R is a $C_{1-4}$ alkyl group - and

the resulting diester of general formula (IV) - wherein R is a $C_{1-4}$ alkyl group - is cyclized into the carboxylic ester of general formula (V) -wherein R has the same meaning as given above - which is then transformed by hydrolysis into the compound of formula (II) and, if desired a salt of the compound of formula (II) is formed.

12. Process, according to claim 11., **characterized by** that the cyclization is carried out in the presence of polyphosphoric acid.

13. The (-)-6-fluoro-1,2,3,4-tetrahydro-2-methyl-quinoline of formula (I) and its salts.

14. The (-)-6-fluoro-1,2,3,4-tetrahydro-2-methyl-quinoline of formula (I) in its essentially optically pure form.

15. The (-)-6-fluoro-1,2,3,4-tetrahydro-2-methyl-quinoline L-(-)-di-para-toluoyl-tartrate acid salt of formula (VI).

16. The (-)-9-fluoro-6,7-dihydro-5-methyl-1-oxo-1$H$,5$H$-pyrido [3,2,1-$i,j$]quinoline-2-carboxylic acid of formula (II) in its essentially optically pure form.

(I)

(II)

(III)

(IV)

COOR

(V)

COOH

CH₃

(VI)

**European Patent Office**    **EUROPEAN SEARCH REPORT**    Application Number

EP 97 89 0092

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,Y | DE 22 64 163 A (RIKER LABORATORIES INC.)<br><br>* page 10, reaction scheme *<br>* claims 1,2 *<br>--- | 1,11,<br>13-16 | C07D215/18<br>C07D471/06<br>C07C69/76<br>C07B57/00<br>//(C07D471/06,<br>  221:00,221:00) |
| D,Y | FR 2 453 647 A (RIKER LABORATORIES, INC.)<br><br>* page 1, line 9 - page 3, line 25 *<br>--- | 1,11,<br>13-16 | |
| Y | EP 0 473 550 A (SANDOZ)<br><br>* examples 5,6,12,13 *<br>--- | 1,11,<br>13-16 | |
| Y | EP 0 297 651 A (DUPHAR INTERNATIONAL RESEARCH B.V.)<br>* example III *<br>--- | 1,11,<br>13-16 | |
| Y | US 4 268 513 A (H. S. SHAPIRO)<br><br>* example 13 *<br>--- | 1,11,<br>13-16 | |
| A | DE 22 21 669 A (PFIZER CORP.)<br>* claims 1,8; examples 1,2 *<br>----- | 1 | **TECHNICAL FIELDS SEARCHED (Int.Cl.6)**<br>C07D<br>C07C<br>C07B |

The present search report has been drawn up for all claims

| Place of search | Date of completion of the search | Examiner |
|---|---|---|
| BERLIN | 1 September 1997 | Hass, C |

EPO FORM 1503 03.82 (P04C01)